# EUROPÄISCHE PATENTSCHRIFT

(11) **EP 1 171 027 B1**
(45) Veröffentlichungstag und Bekanntmachung des Hinweises auf die Patenterteilung: **27.11.2002**
(21) Anmeldenummer: 00938498.3
(22) Anmeldetag: 18.04.2000
(51) Int. Cl.: A61B 5/00

(54) **VORRICHTUNG ZUM AUFNEHMEN EINES THERMOOPTISCHEN BILDES DER WEIBLICHEN BRUST**
DEVICE FOR RECORDING A THERMO-OPTICAL IMAGE OF THE FEMALE BREAST
DISPOSITIF POUR EFFECTUER DES PRISES DE VUE THERMO-OPTIQUES DU SEIN

(30) Priorität: 22.04.1999 DE 29907186 U; 16.06.1999 DE 19927426
(43) Veröffentlichungstag der Anmeldung: 16.01.2002
(73) Patentinhaber: Wehberg, Heinrich, 27299 Langwedel (DE)
(72) Erfinder: Wehberg, Heinrich, 27299 Langwedel (DE)
(74) Vertreter: Goddar, Heinz J., Dr.
(86) Internationale Anmeldenummer: DE0001243
(87) Internationale Veröffentlichungsnummer: WO00064332

(56) Entgegenhaltungen:
- WO-A-81/03418
- WO-A-85/00508
- DE-U- 8 326 341
- US-A- 4 135 497
- US-A- 5 995 865

## Beschreibung

Die vorliegende Erfindung betrifft eine Vorrichtung zum Aufnehmen eines thermooptischen Bildes der weiblichen Brust.

Für die Diagnostik von Mamma-Carcinomen sowie deren Früherkennung im Rahmen der Vorsorgeuntersuchungen wird am häufigsten die Mammographie, also eine Röntgenmethode eingesetzt. Diese Methode ist mit einer - wenn auch geringen - Strahlenbelastung für die Patientin verbunden. Als Ergänzung zur Mammographie steht grundsätzlich die Thermographie zur Verfügung, bei der mit Hilfe der Hauttemperatur an der weiblichen Brust Mamma-Carcinome diagnostiziert werden können.

Im Stand der Technik sind hierzu unterschiedlichste Methoden vorgeschlagen worden. Bei einer Gruppe von Methoden werden die Hauttemperaturen an der weiblichen Brust direkt gemessen und gegebenenfalls elektronisch weiterverarbeitet. So offenbaren z. B. die WO 79/00594 und die US 3,970,074 Vorrichtungen, bei der die Brust an eine mit Temperaturfühlern matrixartig bestückte Platte gedrückt und die Hauttemperatur gemessen wird. Die gemessenen Daten werden elektronisch weiterverarbeitet, wobei bestimmte Temperaturverteilungen an der Haut ein Indiz für ein Mamma-Carcinom sind.

Die US 4,055,166 offenbart einen Büstenhalter, der mit einzelnen Temperaturfühlern bestückt ist. Hiermit wird die Hauttemperatur an den entsprechenden Punkten ständig überprüft. Bestimmte Veränderungen in der Hauttemperatur deuten hier auf das Entstehen von Mamma-Carcinomen hin.

Eine zweite Gruppe der Thermographieverfahren bildet die sogenannte Plattenthermographie. Eine entsprechende Vorrichtung ist in der DE 83 26 341 U1 offenbart. Eine thermographische Platte wird an die weibliche Brust gedrückt. Eine thermographische Beschichtung der Platte nimmt je nach Temperatur unterschiedliche Farben an. Hierdurch werden thermographisch der Verlauf von Gefäßen, die gegenüber anderen Bereichen wärmer sind, optisch sichtbar gemacht. Bestimmte Strukturen bzw. Merkmale deuten auf krankhafte Veränderungen hin. Das eigentliche Diagnostizierverfahren der Plattenthermorgraphie ist genauer im "Atlas der Plattenthermographie" von G. Lauth und G. Mühlberger beschrieben. Dieser Atlas gibt eine erste Einführung für den mit der Plattenthermographie noch unerfahrenen Arzt. Insgesamt erfordert die Durchführung der Plattenthermographie eine große diagnostische Erfahrung des behandelnden Arztes und ist sie bei insbesondere noch unerfahrenen Ärzten mit einer großen Fehlerquote behaftet.

Die WO-A-85 00508 offenbart eine Vorrichtung zum Aufnehmen eines thermooptischen Bildes der weiblichen Brust mit einem Gehäuse, das auf seiner der Brust zugewandten Seite einen Rahmen aufweist, einer thermooptischen Folie, die an dem Rahmen befestigbar ist, einer in dem Gehäuse angeordneten Beleuchtungseinrichtung zum Beleuchten der thermooptischen Folie und einer in dem Gehäuse gegenüber dem Rahmen angeordneten Digitalkamera.

Hiervon ausgehend liegt der Erfindung die Aufgabe zugrunde, eine Vorrichtung bereitzustellen, die eine Diagnostik von pathologischen Veränderungen erleichtert.

Erfindungsgemäß wird diese Aufgabe gelöst durch eine Vorrichtung zum Aufnehmen eines thermooptischen Bildes der weiblichen Brust mit einem Gehäuse, das bis auf seine der Brust zugewandte Seite, auf der es einen Rahmen aufweist, lichtundurchlässig ist, einer thermooptischen Folie, die an dem Rahmen befestigbar ist, einem auf der von der Brust abgewandten Seite des Rahmens angeordneten, durchsichtigen Kühlkasten mit einer auf seiner von der Brust abgewandten Seite angeordneten Antireflexscheibe, der einen Kühlmediumzulauf und einen Kühlmediumablauf aufweist und in seiner Ausgangsstellung nicht mit der Folie in Kontakt steht, einem hinsichtlich der Temperatur eines Kühlmediums einstellbaren Thermostaten. der zur Bildung eines Kühlkreislaufes über Schlauchleitungen mit dem Kühlmediumzulauf und -ablauf in Verbindung steht, einer in dem Gehäuse angeordneten Beleuchtungseinrichtung zum Beleuchten der thermooptischen Folie, einer in dem Gehäuse gegenüber dem Rahmen angeordneten Digitalkamera, einer Klemmeinrichtung zum Klemmen der Brust zwischen zwei Flächen, wobei eine der beiden Flächen von der Folie gebildet wird, einer Kontaktherstelleinrichtung zum Herstellen eines Flächenkontakts zwischen einer der Folie zugewandten Seite des Kühlkastens und der von der Brust abgewandten Seite der Folie, einer Zeitmeßeinrichtung zur Messung der Zeitdauer ab der Kontaktherstellung, und einer mit der Zeitmeßeinrichtung verbundenen Auslöseeinrichtung zum automatischen Auslösen der Digitalkamera nach Ablauf einer vorgebbaren Zeitdauer.

Gemäß einer besonderen Ausführungsform der Erfindung kann vorgesehen sein, daß die Klemmeinrichtung ein mit dem Gehäuse verbundenes, sich parallel zum Rahmen erstreckendes Polster umfaßt, dessen Abstand zum Rahmen veränderbar ist.

Insbesondere kann dabei vorgesehen sein, daß das Polster über eine dazu im rechten Winkel angeordnete Halterung mit dem Gehäuse verbunden ist.

Gemäß einer weiteren besonderen Ausführungsform der Erfindung kann vorgesehen sein, daß der Rahmen viereckig ist und an seinen vier Ecken mittels Stiften an dem Gehäuse gehalten wird, wobei sich Federn konzentrisch zu den Stiften in der Weise erstrecken, daß sich eine an dem Rahmen befestigte Folie in einem unbelasteten Zustand der Federn in einem Abstand von dem Kühlkasten befindet.

Dabei kann eine Arretiereinrichtung zum Arretieren des Rahmens bei Herstellung eines Kontakts zwischen der Folie und dem Kühlkasten nach Zusammendrücken der Federn vorgesehen sein.

Ferner kann ein mehrgelenkiger Arm vorgesehen sein, an dessen einem Ende das Gehäuse angebracht ist.

Eine weitere besondere Ausführungsform der Erfindung ist gekennzeichnet durch einen mit dem anderen Ende des Arms verbundenen Gerätewagen. Dies ermöglicht zum einen einen leichten Transport der Vorrichtung und zum anderen eine leichten Handhabung des Gehäuses vor Ort.

Günstigerweise ist eine weitere besondere Ausführungsform der Erfindung mit einem mit der Digitalkamera verbundenen Bildschirm versehen. Dadurch lassen sich die Bilder auf dem Bildschirm betrachten.

Günstigerweise ist ein Rechner mit einer Tastatur vorgesehen.

Insbesondere kann dabei vorgesehen sein, daß der Rechner die Zeitmeßeinrichtung und die Auslöseeinrichtung enthält.

Eine weitere besondere Ausführungsform der Erfindung ist gekennzeichnet durch eine Speichereinrichtung zum Speichern der mittels der Digitalkamera aufgenommenen digitalen Bilder.

Außerdem ist eine weitere besondere Ausführungsform der Erfindung gekennzeichnet durch eine Ausgabeeinrichtung zum Ausgeben der digitalen Bilder auf einen Drucker oder ein Speichermedium. Bei dem Speichermedium kann es sich z. B. um eine CD-Rom oder eine Diskette handeln.

Eine weitere besondere Ausführungsform der Erfindung ist gekennzeichnet durch eine mit der Digitalkamera verbundene Bildauswerteeinrichtung zur automatischen Auswertung der in dem aufgenommenen Bild enthaltenen Informationen hinsichtlich des Vorliegens einer pathologischen Veränderung.

Günstigerweise weist die Bildauswerteeinrichtung eine Einrichtung zum Vergleichen des aktuellen Bildes mit früheren Bildern derselben Brust bzw. mit Referenzbildern auf.

Außerdem kann vorgesehen sein, daß die Bildauswerteeinrichtung eine Einrichtung zum Absuchen eines Bildes nach für Mamma-Carcinome charakteristischen Strukturen bzw. Merkmalen aufweist.

Schließlich ist eine weitere besondere Ausführungsform der Erfindung dadurch gekennzeichnet, daß der Bildschirm in zwei Teile unterteilt ist, wobei ein Fenster zur Anzeige des aktuellen Bildes und das andere Fenster zur Anzeige eines früheren Bildes derselben Brust oder eines Referenzbildes dient.

Der Erfindung liegt die überraschende Erkenntnis zugrunde, daß durch den Kühlkasten in Kombination mit dem Kühlkreislauf, die Beleuchtungseinrichtung und die Klemmeinrichtung eine Standardisierung der thermooptischen Bilder möglich ist. Dies bedeutet, daß für ein und dieselbe Patientin immer dieselben Aufnahmebedingungen reproduzierbar sind, so daß pathologische Veränderungen vom behandelnden Arzt leicht erkennbar sind, das heißt leichter eine zuverlässigere Diagnose vorgenommen werden kann. Darüber hinaus ermöglichen standardisierte thermooptische Bilder eine automatische Diagnostik.

Weitere Merkmale und Vorteile der Erfindung ergeben sich aus den Ansprüchen und aus der nachstehenden Beschreibung, in der ein Ausführungsbeispiel anhand der schematischen Zeichnungen im einzelnen erläutert ist. Dabei zeigt:
Figur 1 eine schematische Seitenansicht eines Teils einer besonderen Ausführungsform einer Vorrichtung zum Aufnehmen eines thermooptischen Bildes der weiblichen Brust;
Figur 2 einen weiteren Teil der Vorrichtung von Figur 1; und
Figur 3 schematisch die gesamte Vorrichtung von Figur 1 im Einsatz bei einer Patientin.

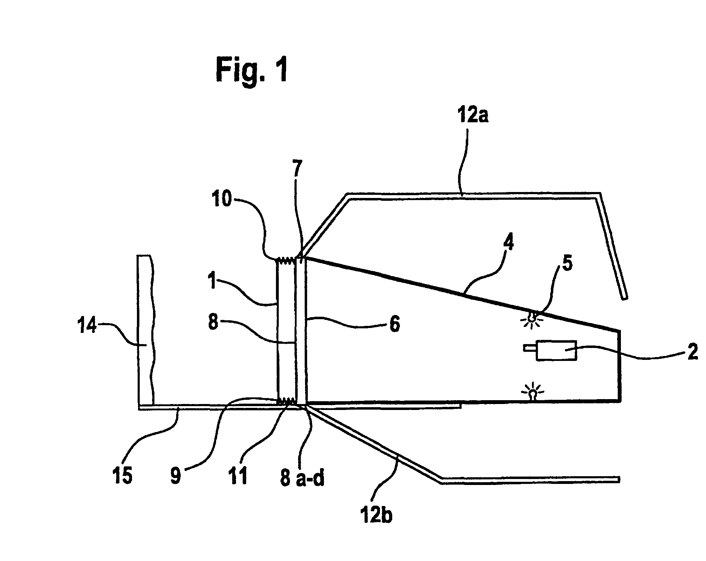

Figur 1 zeigt schematisch einen Teil einer besonderen Ausführungsform des erfindungsgemäßen Vorrichtung in Seitenansicht. Diese umfaßt ein Gehäuse 4, das zur Standardisierung der thermooptischen Bilder bis auf seine der Brust zugewandte Seite, auf der es einen Rahmen 9 aufweist, lichtundurchlässig ist. Damit ist die Voraussetzung für eine von Außenreflexen freie Aufnahme gegeben. Der Rahmen 9 dient zur Aufnahme einer thermooptischen Folie 1. Auf der in das Gehäuseinnere zeigenden Seite des Rahmens 9 ist ein durchsichtiger Kühlkasten 7 mit einer auf seiner zum Gehäuseinneren zeigenden Seite angeordneten Antireflexscheibe 6 angeordnet. Die Antireflexscheibe 6 bildet gleichzeitig die Rückwand des Kühlkastens 7. Die auf den schmalen Rahmen 9 gespannte Folie 1 befindet sich in einem Abstand von 2 bis 3 Zentimetern vor der Vorderwand 8 des Kühlkastens 7. Der viereckige Rahmen 9 wird an den vier Ecken des Gehäuses 4 durch Stifte 10 aus Stahl gehalten, wobei der Abstand der Folie 1 zur Vorderwand des Kühlkastens 7 durch vier Federn 11 gewährleistet ist. Die Federn 11 sind über die Stifte 10 gestülpt und im entspannten Zustand so lang, daß sie die Folie 1 auf den besagten Abstand zur Vorderwand 8 des Kühlkastens 7 halten. Nach Aufsetzen der Folie 1 auf die zu untersuchende Brust ist eine Korrektur der Position der Folie 1 durchaus möglich, ohne daß bereits ein Flächenkontakt zwischen der Folie 1 und der Vorderwand 8 des Kühlkastens 7 stattgefunden hat.

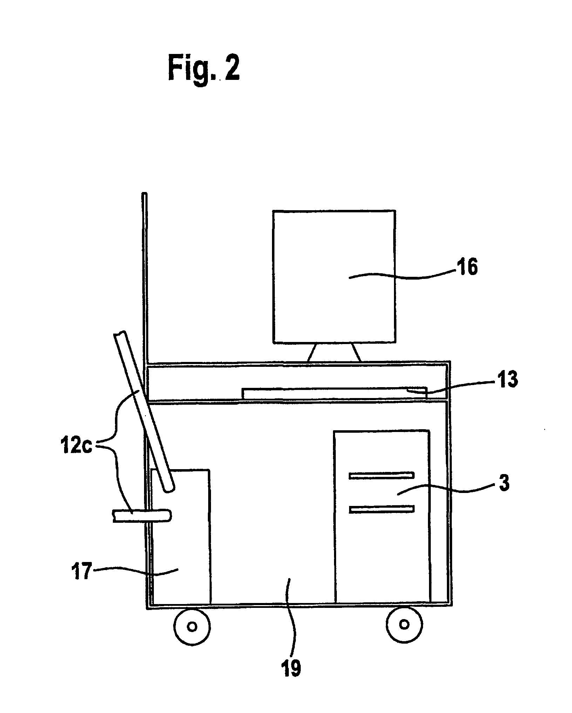

Der Kühlkasten 7 weist einen Kühlmediumzulauf 12a und einen Kühlmediumablauf 12b auf, über die er mittels Schlauchleitungen 12c in einem Kreislauf mit einem Thermostat 17 (siehe Figur 2) verbunden ist. Als Kühlmedium dient Wasser. Ein ständiger Wasserrundlauf und eine konstante Wassertemperatur sorgen für eine Konstanz der Temperatur der Folie 1. Dabei sollte die eingestellte Kühlung - immer bezogen auf die Wärmeempfindlichkeit der Folie 1 - konstant sein. Der Grad der Kühlung ist über eine Tastatur 13 (siehe Figur 2) eines Rechners 3 (siehe Figur 2) von Hand einstellbar.

Weiterhin befindet sich in dem Gehäuse 4 eine Digitalkamera 2 gegenüber dem Rahmen 9. Eine Beleuchtungseinrichtung in Form von zwei Lampen 5 dient zum Beleuchten der Folie 1 und ist so ausgerichtet, daß die Aufnahmen mit der Digitalkamera optimiert werden. Die Lampen 5 tragen ebenfalls zur Standardisierung bei.

Ein Polster 14 läßt sich zum sanften Klemmen der Brust zwischen der Folie 1 und eben dem Polster 14 über eine Halterung 15 in Figur 1 in horizontaler Ebene hin und her bewegen. Dies trägt ebenfalls zur Standardisierung bei.

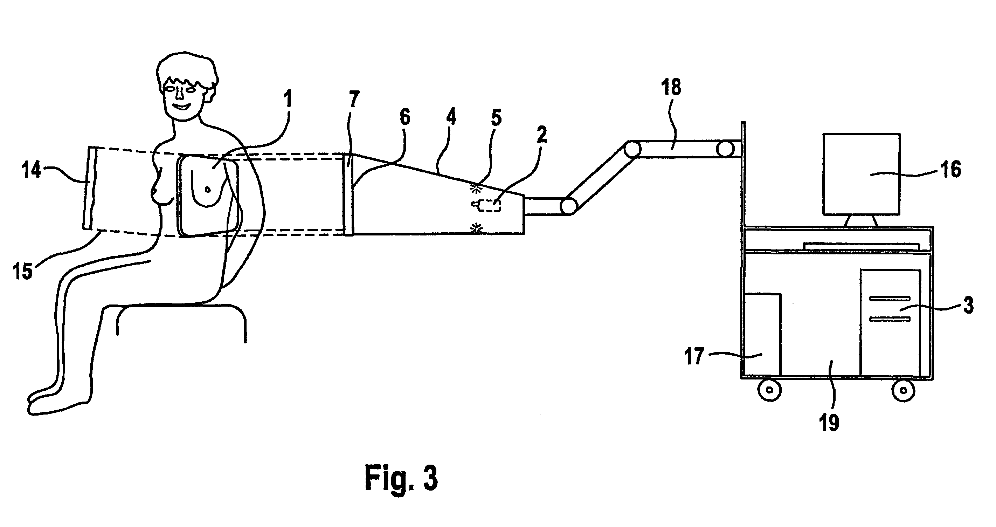

Figur 2 zeigt schematisch einen weiteren Teil der Vorrichtung von Figur 1. In einem Gerätewagen 19 befinden sich ein Thermostat 17, ein Rechner 3 mit einer Tastatur 13 und ein Bildschirm 16. An dem Gerätewagen 19 ist ein hier nicht, aber in Figur 3 gezeigter mehrgelenkigen Arm 18 befestigt, an dessen anderem Ende das Gehäuse 4 befestigt ist. Der Arm erlaubt eine Beweglichkeit und ist arretierbar. In seinem Inneren verlaufen die Versorgungsleitungen (nicht gezeigt) für die Digitalkamera 2, die Kühlung des Kühlkastens 7 und die Beleuchtungseinrichtungen 5. Nachfolgend soll nun der Einsatz des Systems anhand von Figur 3 beschrieben werden, in der die Folie 1 zu Darstellungszwecken um ca. 90° nach vorne gedreht ist:

Durch Auflegen einer thermooptischen Folie 1 auf die weibliche Brust entsteht auf der Folie ein Wärmemuster in verschiedenen Farben entsprechend den arealen Wärmezuständen der Haut. Dieses Wärmemuster ist nach Entfernen der Folie von der Brust reversibel. Durch Zuführen von Kälte kann die Farbgebung auf der Folie 1 minimiert werden. Die sich darstellenden Farbmuster sind schärfer konturiert und zur schwarzen Umgebung kontrastreicher. Durch Zufuhr von Kälte soll in erster Linie die Hautwärme "weggekühlt" werden und sollen die Peaks der aus tiefer gelegenen Wärmequellen abgegebenen Wärme zur Darstellung kommen.

Die Aufnahmepositionen entsprechen denjenigen der Mammographie, allerdings mit dem Unterschied, daß pro Brust bei normal großen Mammae zwei seitliche und zwei craniocaudale Aufnahmen angefertigt werden. Dies geschieht aus dem Grunde, daß bei der "Vascugraphie" die abgestrahlte Wärme gemessen wird und durch die Messung von beiden Seiten eine größere Sensivität erreicht werden kann. Die Brust wird nach Auflegen der Folie 1 auf die Brust mit dem weichen Polster 14, das von der Patientin selber in Position gebracht werden kann, gehalten. Dies hat den Vorteil, daß die unangenehme Assoziation zur "Quetschuntersuchung" der Mammographie entfällt. Da allerdings bei konstanter Temperatur des Kühlmediums und gleichbleibender Wärmeempfindlichkeit der Folie während einer Untersuchung die Dauer der Kühlung nicht ohne Bedeutung, andererseits auch hier eine Standardisierung zu verlangen ist, muß der Zeitpunkt des Anfangs und des Endes des Kühlvorgangs definiert sein. Dies wird dadurch erreicht, daß in dem Moment, in dem die Folie 1 durch sanften Druck gegen die Spannung der Federn 10 an den Kühlkasten 7 gepreßt wird und eine Arretierung (nicht gezeigt) einschnappt, eine Zeitmeßeinrichtung (nicht gezeigt) mit dem Messen der Kühlzeitdauer beginnt und am Ende einer vorgebbaren Kühlzeitdauer mittels einer Auslöseeinrichtung (nicht gezeigt) die Digitalkamera 2 automatisch ausgelöst wird. Nach jeder Aufnahme wird das Polster 14 über eine Halterung 15 mit Gasfederdruck (nicht gezeigt) in seine Ausgangsstellung gebracht. Durch Standardisierung dieser Einstellungen erreicht man eine maximale Reproduzierbarkeit. Alle Einstellungen sind während der Aufnahmen auf dem Bildschirm 16 kontrollierbar. Das Maß der Folie entspricht dem eines Mammographie-Films in der Größe 18 mal 24 Zentimeter. Bei Ersetzen der Folie durch ein fertiges Mammographie-Bild (z. B. eines positiven Falles) und homogener Durchleuchtung des Films von außen in Richtung der Digitalkamera 2 kann man eine einfache und preiswerte Form der digitalisierten Abspeicherung der zur Patientin passenden Mammographie-Aufnahme erreichen. Das deckungsgleiche Übereinanderlegen des Wärmebildes und des Mammographie-Bildes auf dem Bildschirm kann dem Diagnostiker wertvolle Hinweise geben.

Das von der Digitalkamera 2 aufgenommene thermooptische Bild wird in den Rechner 3 übertragen. Im Rechner 3 erfolgt in einer Bildauswerteeinrichtung eine automatische Auswertung des Bildes mittels einer speziellen Software.

Mit der vorangehend beschriebenen Vorrichtung wird die Deutung der thermooptischen Bilder nicht mehr dem untersuchenden Arzt überlassen, sondern von einer Bildauswerteeinrichtung durchgeführt. Diese erkennt aufgrund des thermographisch gewonnenen Gefäßverlaufs und der Form der Gefäße der Patientin anhand von Referenzdaten pathologische Veränderungen. Dies bietet sich insbesondere bei Erstuntersuchungen einer Patientin an. Wenn jedoch bereits Voraufnahmen vorliegen, kann ein Vergleich der aktuellen Aufnahme mit der Voraufnahme durchgeführt werden. Dieser Vorgehensweise liegt die Erkenntnis zugrunde, daß der Gefäßverlauf in der weiblichen Brust gleichsam wie ein Fingerabdruck für jede Frau individuell ist und unverändert bleibt. Kommt es zu Veränderungen im Gefäßverlauf und mithin im thermooptischen Bild, deutet dies auf eine pathologische Veränderung hin, die sodann weiter, beispielsweise durch Biopsie, untersucht werden kann.

Die in der vorstehenden Beschreibung, in den Zeichnungen sowie in den Ansprüchen offenbarten Merkmale der Erfindung können sowohl einzeln als auch in beliebigen Kombinationen für die Verwirklichung der Erfindung in ihren verschiedenen Ausführungsformen wesentlich sein.

## Patentansprüche

1. Vorrichtung zum Aufnehmen eines thermooptischen Bildes der weiblichen Brust mit
- mit einem Gehäuse (4), das bis auf seine der Brust zugewandte Seite, auf der es einen Rahmen (9) aufweist, lichtundurchlässig ist,
- einer thermooptischen Folie (1), die an dem Rahmen (9) befestigbar ist,
- einem auf der von der Brust abgewandten Seite des Rahmens (9) angeordneten, durchsichtigen Kühlkasten (7) mit einer auf seiner von der Brust abgewandten Seite angeordneten Antireflexscheibe (6), der einen Kühlmediumzulauf (12a) und einen Kühlmediumablauf (12b) aufweist und in seiner Ausgangsstellung nicht mit der Folie (1) in Kontakt steht,
- einem hinsichtlich der Temperatur eines Kühlmediums einstellbaren Thermostaten (17), der zur Bildung eines Kühlkreislaufes über Schlauchleitungen (12c) mit dem Kühlmediumzulauf (12a) und -ablauf (12b) in Verbindung steht,
- einer in dem Gehäuse (4) angeordneten Beleuchtungseinrichtung zum Beleuchten der thermooptischen Folie (1),
- einer in dem Gehäuse (4) gegenüber dem Rahmen (9) angeordneten Digitalkamera (2),
- einer Klemmeinrichtung zum Klemmen der Brust zwischen zwei Flächen, wobei eine der beiden Flächen von der Folie (1) gebildet wird,
- einer Kontaktherstelleinrichtung zum Herstellen eines Flächenkontakts zwischen einer der Folie (1) zugewandten Seite des Kühlkastens (7) und der von der Brust abgewandten Seite der Folie (1),
- einer Zeitmeßeinrichtung zur Messung der Zeitdauer ab der Kontaktherstellung, und
- einer mit der Zeitmeßeinrichtung verbundenen Auslöseeinrichtung zum automatischen Auslösen der Digitalkamera (2) nach Ablauf einer vorgebbaren Zeitdauer.

2. Vorrichtung nach Anspruch 1, **dadurch gekennzeichnet, daß** die Klemmeinrichtung ein mit dem Gehäuse (4) verbundenes, sich parallel zum Rahmen erstreckendes Polster (14) umfaßt, dessen Abstand zum Rahmen (9) veränderbar ist.

3. Vorrichtung nach Anspruch 2, **dadurch gekennzeichnet, daß** das Polster (14) über eine dazu im rechten Winkel angeordnete Halterung (15) mit dem Gehäuse (4) verbunden ist.

4. Vorrichtung nach einem der Ansprüche 1 bis 3, **dadurch gekennzeichnet, daß** der Rahmen (9) viereckig ist und an seinen vier Ecken mittels Stiften (10) an dem Gehäuse (4) gehalten wird, wobei sich Federn (11) konzentrisch zu den Stiften (10) in der Weise erstrecken, daß sich eine an dem Rahmen (9) befestigte Folie (1) in einem unbelasteten Zustand der Federn (11) in einem Abstand von dem Kühlkasten (7) befindet.

5. Vorrichtung nach Anspruch 4 **gekennzeichnet durch** eine Arretiereinrichtung zum Arretieren des Rahmens (9) bei Herstellung eines Kontakts zwischen der Folie (1) und dem Kühlkasten (7) nach Zusammendrücken der Federn (11).

6. Vorrichtung nach einem der vorangehenden Ansprüche, **gekennzeichnet durch** einen mehrgelenkigen Arm (18), an dessen einem Ende das Gehäuse (4) angebracht ist.

7. Vorrichtung nach Anspruch 6, **gekennzeichnet durch** einen mit dem anderen Ende des Arms (18) verbundenen Gerätewagen (19).

8. Vorrichtung nach einem der vorangehenden Ansprüche, **gekennzeichnet durch** einen mit der Digitalkamera (2) verbundenen Bildschirm (16).

9. Vorrichtung nach einem der vorangehenden Ansprüche, **gekennzeichnet durch** einen Rechner (3) mit einer Tastatur (13).

10. Vorrichtung nach Anspruch 9, **dadurch gekennzeichnet, daß** der Rechner (3) die Zeitmeßeinrichtung und die Auslöseeinrichtung enthält.

11. Vorrichtung nach einem der vorangehenden Ansprüche, **gekennzeichnet durch** eine Speichereinrichtung zum Speichern der mittels der Digitalkamera (2) aufgenommenen digitalen Bilder.

12. Vorrichtung nach einem der vorangehenden Ansprüche, **gekennzeichnet durch** eine Ausgabeeinrichtung zum Ausgeben der digitalen Bilder auf einen Drucker oder ein Speichermedium.

13. Vorrichtung nach einem der vorangehenden Ansprüche, **gekennzeichnet durch** eine mit der Digitalkamera (2) verbundene Bildauswertereinrichtung zur automatischen Auswertung der in dem aufgenommenen Bild enthaltenen Informationen hinsichtlich des Vorliegens einer pathologischen Veränderung.

14. Vorrichtung nach Anspruch 13, **dadurch gekennzeichnet, daß** die Bildauswertereinrichtung eine Einrichtung zum Vergleichen des aktuellen Bildes mit früheren Bildern derselben Brust bzw. mit Referenzbildern aufweist.

15. Vorrichtung nach Anspruch 13, **dadurch gekennzeichnet, daß** die Bildauswertereinrichtung eine Einrichtung zum Absuchen eines Bildes nach für Mamma-Carcinome charakteristischen Strukturen bzw. Merkmalen aufweist.

16. Vorrichtung nach Anspruch 14 oder 15, jeweils wenn abhängig von Anspruch 8, **dadurch gekennzeichnet, daß** der Bildschirm (16) in zwei Fenster unterteilt ist, wobei ein Fenster zur Anzeige des aktuellen Bildes und das andere Fenster zur Anzeige eines früheren Bildes derselben Brust oder eines Referenzbildes dient.

## Claims

1. Apparatus for taking a thermo-optical image of the female breast with
- a housing (4) which is opaque except for its side facing the breast, on which it has a frame (9),
- a thermo-optical film (1) which is adapted to be fixed to the frame (9),
- a transparent cooling box (7) which is disposed on the side of the frame (9) remote from the breast and which has an anti-reflection pane (6) disposed on its side remote from the breast and having a cooling medium inlet (12a) and cooling medium outlet (12b), said pane (6) not being in contact with the film (1) in its starting position,
- a thermostat (17) which is adjustable in respect of the temperature of a cooling medium and which, for the purpose of forming a cooling circuit, is connected via hoses (12c) to the cooling medium inlet (12a) and outlet (12b),
- a lighting device disposed in the housing (4) to illuminate the thermo-optical film (1),
- a digital camera (2) disposed opposite the frame (9) in the housing (4),
- a clamping device for clamping the breast between two surfaces, one of the two surfaces being formed by the film (1),
- a contact-making device for making a surface contact between a side of the cooling box (7) facing the film (1) and the side of the film (1) remote from the breast,
- a time measuring device for measuring the time elapsing from contact-making and
- a triggering device connected to the time measuring device for automatically triggering the digital camera (2) after expiry of a predeterminable time.

2. Apparatus according to claim 1, **characterised in that** the clamping device comprises a cushion (14) which is connected to the housing (4), extends parallel to the frame, and the distance of which from the frame (9) is variable.

3. Apparatus according to claim 2, **characterised in that** the cushion (14) is connected to the housing (4) via a mount (15) disposed at a right angle thereto.

4. Apparatus according to any one of claims 1 to 3, **characterised in that** the frame (9) is rectangular and is held on the housing (4) by means of pins (10) at its four corners, springs (11) extending concentrically to the pins (10) in such manner that a film (1) fixed on the frame (9) is at a distance from the cooling box (7) when the springs (11) are in a non-loaded state.

5. Apparatus according to claim 4, **characterised by** a locking device for locking the frame (9) when contact is made between the film (1) and the cooling box (7) after compression of the springs (11).

6. Apparatus according to any one of the preceding claims, **characterised by** a multi-jointed arm (18) at one end of which the housing (4) is mounted.

7. Apparatus according to claim 6, **characterised by** an equipment trolley (19) connected to the other end of the arm (18).

8. Apparatus according to any one of the preceding claims, **characterised by** a monitor (16) connected to the digital camera (2).

9. Apparatus according to any one of the preceding claims, **characterised by** a computer (3) with a keyboard (13).

10. Apparatus according to claim 9, **characterised in that** the computer (3) contains the time measuring device and the triggering device.

11. Apparatus according to any one of the preceding claims, **characterised by** a memory device for storing the digital images taken by means of the digital camera (2).

12. Apparatus according to any one of the preceding claims, **characterised by** an outputting device for outputting the digital images to a printer or a storage medium.

13. Apparatus according to any one of the preceding claims, **characterised by** an image evaluating device connected to the digital camera (2) for automatic evaluation of the information contained in the recorded image in respect of the presence of a pathological change.

14. Apparatus according to claim 13, **characterised in that** the image evaluating device comprises a device for comparing the current image with earlier images of the same breast or with reference images.

15. Apparatus according to claim 13, **characterised in that** the image evaluating device comprises a device for examining an image for structures or features characteristic of breast carcinoma.

16. Apparatus according to claim 14 or 15 each when appended to claim 8, **characterised in that** the screen of the monitor (16) is divided into two windows, one window being used to display the current image and the other window being used to display an earlier image of the same breast or a reference image.

## Revendications

1. Dispositif pour enregistrer une image thermooptique du sein féminin, comprenant
- un coffret (4) qui est opaque à l'exception de son côté tourné vers le sein, sur lequel il comporte un cadre (9),
- une feuille thermooptique (1) qui peut être fixée au cadre (9),
- un caisson de refroidissement transparent (7), lequel est disposé sur le côté du cadre (9) situé à l'opposé du sein, comporte une vitre antireflet (6) disposée sur son côté situé à l'opposé du sein, comporte une arrivée d'agent de refroidissement (12a) et une sortie d'agent de refroidissement (12b) et, dans sa position initiale, n'est pas en contact avec la feuille (1),
- un thermostat (17) qui est réglable en fonction de la température d'un agent de refroidissement et qui, pour former un circuit de refroidissement, est relié par des conduites souples (12c) à l'arrivée (12a) et à la sortie (12b) d'agent de refroidissement,
- un dispositif d'éclairage disposé dans le coffret (4) pour éclairer la feuille thermooptique (1),
- une caméra numérique (2) disposée dans le coffret (4) en face du cadre (9),
- un dispositif de serrage pour serrer le sein entre deux surfaces, une des deux surfaces étant formée par la feuille (1),
- un dispositif de mise en contact pour créer un contact de surface entre le côté du caisson de refroidissement (7) tourné vers la feuille (1) et le côté de la feuille (1) situé à l'opposé du sein,
- un dispositif de chronométrage pour mesurer la durée à partir de la mise en contact, et
- un dispositif de déclenchement relié au dispositif de chronométrage pour déclencher automatiquement la caméra numérique (2) après écoulement d'une durée prédéfinie.

2. Dispositif selon la revendication 1, **caractérisé en ce que** le dispositif de serrage comprend un rembourrage (14) qui est relié au coffret (4) et s'étend parallèlement au cadre et dont la distance au cadre (9) est modifiable.

3. Dispositif selon la revendication 2, **caractérisé en ce que** le rembourrage (14) est relié au coffret (4) par l'intermédiaire d'un support (15) disposé à angle droit par rapport à lui.

4. Dispositif selon une des revendications 1 à 3, **caractérisé en ce que** le cadre (9) est quadrangulaire et est maintenu au coffret (4) dans ses quatre angles au moyen de broches (10), des ressorts (11) s'étendant concentriquement aux broches (10), de façon que, à l'état relâché des ressorts (11), une feuille (1) fixée au cadre (9) se trouve à distance du caisson de refroidissement (7).

5. Dispositif selon la revendication 4, **caractérisé par** un dispositif d'arrêt pour arrêter le cadre (9) lors de l'établissement d'un contact entre la feuille (1) et le caisson de refroidissement (7) après compression des ressorts (11).

6. Dispositif selon une des revendications précédentes, **caractérisé par** un bras pluri-articulé (18), à l'une des extrémités duquel est disposé le coffret (4).

7. Dispositif selon la revendication 6, **caractérisé par** un chariot technique (19) relié à l'autre extrémité du bras (18).

8. Dispositif selon une des revendications précédentes, **caractérisé par** un écran (16) relié à la caméra numérique (2).

9. Dispositif selon une des revendications précédentes, **caractérisé par** un ordinateur (3) avec un clavier (13).

10. Dispositif selon la revendication 9, **caractérisé en ce que** l'ordinateur (3) contient le dispositif de chronométrage et le dispositif de déclenchement.

11. Dispositif selon une des revendications précédentes, **caractérisé par** un dispositif de mémorisation pour mémoriser les images numériques enregistrées au moyen de la caméra numérique (2).

12. Dispositif selon une des revendications précédentes, **caractérisé par** un dispositif d'édition pour éditer les images numériques sur une imprimante ou sur un support de mémorisation.

13. Dispositif selon une des revendications précédentes, **caractérisé par** un dispositif d'analyse d'image relié à la caméra numérique (2) pour analyser automatiquement les informations contenues dans l'image enregistrée en recherchant la présence d'une modification pathologique.

14. Dispositif selon la revendication 13, **caractérisé en ce que** le dispositif d'analyse d'image comporte un dispositif pour comparer l'image instantanée à des images antérieures du même sein, respectivement à des images de référence.

15. Dispositif selon la revendication 13, **caractérisé en ce que** le dispositif d'analyse d'image comporte un dispositif pour rechercher, dans une image, des structures, respectivement des signes caractéristiques du cancer du sein.

16. Dispositif selon la revendication 14 ou 15, respectivement en fonction de la revendication 8, **caractérisé en ce que** l'écran (16) est divisé en deux fenêtres, l'une des fenêtres servant à visualiser l'image instantanée et l'autre fenêtre servant à visualiser une image antérieure du même sein ou une image de référence.
